# DEMANDE DE BREVET EUROPEEN

(11) **EP 4 684 867 A2**
(43) Date de publication de la demande: **28.01.2026**
(21) Numéro de dépôt: 25190022.1
(22) Date de dépôt: 16.07.2025
(51) Int. Cl.: B01F 31/40, B01F 31/441, B01F 33/501, B01F 35/75, B01F 101/20, B01F 101/28

(54) **DISPOSITIF POUR LE MELANGE ET L'INJECTION D'UNE COMPOSITION, NOTAMMENT UN CIMENT OSSEUX**

(30) Priorité: 25.07.2024 FR 2408243
(71) Demandeur: Histone, 31400 Toulouse (FR)
(72) Inventeur: NAVARRO NIXON, Elena Julia, 31200 Toulouse (FR); SAHRAOUI, Nourédine, 31400 Toulouse (FR)
(74) Mandataire: Santarelli

(57) **Abrégé**

L'invention concerne un dispositif (100) pour le mélange et l'injection d'une composition comprenant :
- Un réservoir (200) délimitant une chambre interne (204), et comportant une ouverture taraudée (206),
- Un ensemble piston (600) comportant une tige filetée (610), une poignée (650) et une tête de piston (630),
la tige filetée (610) :
∘ coopérant avec l'ouverture taraudée (206) du réservoir (200),
∘ comportant un canal interne longitudinal (613) débouchant,

la tête de piston (630) :
∘ étant agencée dans la chambre interne (204),
∘ comportant un orifice traversant (632) dans la continuité du canal interne longitudinal,

- Un ensemble mélangeur (400) comportant une tige (410), une poignée (450) et une palette de mélange (430),
la tige (410) s'étendant dans le canal interne longitudinal de la tige filetée et dans l'orifice traversant de la tête de piston,
la palette de mélange étant agencée dans la chambre interne (204).

## Description

### Domaine technique de l'invention

La présente invention se rapporte à un dispositif pour le mélange et l'injection d'une composition.

L'objet de l'invention trouve une application particulièrement avantageuse dans le domaine de la chirurgie osseuse. La composition est un ciment osseux, notamment de nature à assurer une reconstruction osseuse.

### Technique antérieure

Dans de nombreux domaines, il peut être nécessaire que des composants, de même phase ou de phases différentes, soient mélangés peu de temps avant leur utilisation, par exemple parce que la composition obtenue est instable ou parce que la composition obtenue se solidifie dans un temps relativement court.

Dans le domaine du bâtiment, par exemple, il est connu de recourir à l'injection d'une composition dans des fissures au niveau de parois de bâtiments en vue de les colmater. La composition, de type colle, est généralement à prise rapide.

Dans le domaine médical, plus particulièrement, il est connu de recourir à l'injection d'une composition, communément appelé ciment osseux, dans un os à traiter afin du consolider. Les opérations chirurgicales y recourant, telles que par exemple les opérations de cimentoplastie, de vertébroplastie, d'arthroplastie, permettent de traiter un traumatisme, tel qu'une fracture osseuse, ou encore une dégradation de l'os due à une maladie. Il est également connu de recourir à l'injection de ciment osseux par exemple pour la pose d'implants, tels que les articulations de la hanche.

Les ciments osseux classiquement utilisés sont composés de deux composants, généralement une poudre et un liquide, qui sont mélangés pour obtenir une composition homogène, sous la forme d'une pâte plus ou moins épaisse. Les ciments osseux sont également à prise rapide, généralement de l'ordre d'une quinzaine de minutes. Il est donc important de réaliser le mélange des composants et d'injecter le ciment osseux dans la zone à traiter dans un temps relativement court.

Quelques dispositifs ont été développés pour permettre de réaliser pour certains le mélange des composants constituant le ciment osseux et pour d'autres l'injection du ciment osseux obtenu dans l'os à traiter.

Cependant, ces dispositifs présentent de nombreux inconvénients. Certains dispositifs se révèlent être de réalisation complexe, notamment car ils sont composés de nombreuses pièces à assembler, et leur utilisation nécessite en conséquence beaucoup de manipulation de la part d'un opérateur. D'autres dispositifs sont de grande dimension et/ou présentant un poids non négligeable, ce qui rend difficile leur manipulation par l'opérateur. Par ailleurs, l'opérateur doit manipuler plusieurs dispositifs, un dispositif pour le mélange des composants et un dispositif pour l'injection du ciment osseux. La manipulation de plusieurs dispositifs successifs génère une perte de temps non négligeable alors que le ciment osseux est à prise rapide. De plus, le temps opératoire, et donc le temps d'anesthésie, s'en trouvent rallongés en conséquence. Le stockage de ces dispositifs et leur recyclage soulève également une problématique non négligeable.

Enfin, les dispositifs présents sur le marché ne sont pas tous adaptés à des ciments osseux du type pâteux, voire très pâteux, c'est-à-dire présentant une haute viscosité. Par viscosité haute, on entend une viscosité d'au moins 80 MPa.s. Les dispositifs pour le mélange des composants sont, pour certains, dans l'incapacité de mélanger des composants en vue d'obtenir un tel ciment osseux tandis que les dispositifs pour l'injection du ciment osseux sont, pour beaucoup, dans l'incapacité d'injecter un tel ciment osseux très pâteux.

### Présentation de l'invention

La présente invention vise à remédier aux inconvénients précités.

A cet effet, il est proposé par la présente invention un dispositif pour le mélange et l'injection d'une composition, dit dispositif, comprenant :
- un réservoir comportant une paroi cylindrique, une première extrémité et une seconde extrémité, délimitant une chambre interne, et d'axe longitudinal,
   le réservoir comportant, au niveau de sa première extrémité, une ouverture taraudée,
   le réservoir comportant, au niveau de sa seconde extrémité, un orifice de sortie,
- un ensemble piston comportant une tige filetée, une poignée située à une première extrémité de la tige filetée et une tête de piston située à une seconde extrémité de la tige filetée,
   la tige filetée :
   ∘ coopérant avec l'ouverture taraudée du réservoir,
   ∘ s'étendant selon l'axe longitudinal du réservoir,
   ∘ comportant un canal interne longitudinal débouchant à ses deux extrémités longitudinales,
   la tête de piston :
   ∘ étant agencée dans la chambre interne du réservoir,
   ∘ ayant une section de forme complémentaire à la forme de la section de la paroi cylindrique du réservoir,
   ∘ comportant un orifice traversant dans la continuité du canal interne longitudinal de la tige filetée,
- un ensemble mélangeur comportant une tige, une poignée située à une première extrémité de la tige et une palette de mélange située à une seconde extrémité de la tige,
   la tige de l'ensemble mélangeur s'étendant dans le canal interne longitudinal de la tige filetée et dans l'orifice traversant de la tête de piston de l'ensemble piston,
   la palette de mélange étant agencée dans la chambre interne du réservoir.

L'ensemble mélangeur est destiné à permettre à un opérateur de mélanger les composants préalablement introduits dans la chambre interne du réservoir afin d'obtenir la composition. L'ensemble piston est destiné à permettre à l'opérateur de transférer la composition obtenue dans la chambre interne du réservoir, après mélange des composants par l'ensemble mélangeur, hors de ladite chambre interne, via l'orifice de sortie.

L'ensemble mélangeur est utilisé pendant une phase, dite de mélange. L'ensemble piston est utilisé pendant une phase, dite d'injection.

Pendant la phase de mélange, la tête de piston de l'ensemble piston est placée au niveau de la première extrémité du réservoir. L'opérateur manipule uniquement la poignée de l'ensemble mélangeur en appliquant notamment un mouvement de translation à l'ensemble mélangeur. Ce mouvement de translation permet le déplacement de la palette de mélange en translation dans la chambre interne du réservoir, entre la première extrémité et la seconde extrémité dudit réservoir. Le déplacement de la palette de mélange dans la chambre interne du réservoir permet ainsi le mélange des composants entre eux jusqu'à l'obtention de la composition voulue.

Pendant la phase d'injection, l'opérateur manipule uniquement la poignée de l'ensemble piston et la fait tourner en rotation pour visser la tige filetée dans l'ouverture taraudée du réservoir, entrainant le déplacement en translation de la tête de piston vers la seconde extrémité du réservoir, poussant la composition obtenue à travers l'orifice de sortie du réservoir.

Le dispositif selon l'invention permet ainsi avantageusement à la fois le mélange des composants constituant la composition, via l'ensemble mélangeur, et d'autre part l'injection de la composition, via l'ensemble piston.

De par le fait que l'ensemble mélangeur est imbriqué dans l'ensemble piston, le dispositif selon l'invention se révèle peu volumineux, notamment en vue de son stockage avant utilisation.

De par le peu de pièces le constituant, le dispositif selon l'invention nécessite moins de manipulation de la part de l'opérateur et se révèle facile d'utilisation. L'opérateur gagne ainsi du temps par rapport à l'utilisation des dispositifs existants, et peut ainsi utiliser des ciments à prise rapide.

Grâce à l'engagement de tige filetée de l'ensemble piston dans l'ouverture taraudée du réservoir, le dispositif selon l'invention permet avantageusement d'injecter aisément la composition, même lorsque celle-ci est de type pâteux, c'est-à-dire une composition présentant une forte viscosité, sans se rompre.

La composition peut être obtenue, sans que cela soit limitatif, à partir du mélange :
- d'une poudre et d'un liquide, ou
- de deux liquides,
- de deux gels,
- d'un gel et d'un liquide.

Le dispositif selon l'invention peut être utilisé dans de nombreux domaines d'application, tels que par exemple le domaine du bâtiment. Ainsi, le dispositif permet le mélange et l'injection d'une composition dans des fissures au niveau de parois de bâtiments en vue de les colmater.

Le dispositif selon l'invention est particulièrement adapté pour être utilisé en milieu médical, notamment pour des opérations de chirurgie osseuse, telles que la cimentoplastie, la vertébroplastie, l'arthroplastie, en vue de consolider un os ou une vertèbre. Le fait que le dispositif puisse mélanger et injecter un ciment osseux de type pâteux présente l'avantage que le temps opératoire, et donc le temps d'anesthésie, sont réduits, diminuant les risques pour les patients. De plus, le recours à un ciment osseux de type pâteux diminue le risque de fuite du ciment osseux dans l'organisme du patient.

Selon des modes particuliers de réalisation, l'invention répond en outre aux caractéristiques suivantes, mises en œuvre séparément ou en chacune de leurs combinaisons techniquement opérantes.

Dans des modes particuliers de réalisation, pour permettre l'introduction des composants dans la chambre interne du réservoir, le réservoir est réalisé en deux parties amovibles, une première partie comportant l'ouverture taraudée du réservoir et une seconde partie comportant l'orifice de sortie du réservoir, la première partie formant un couvercle pour la deuxième partie.

Dans des modes particuliers de réalisation, pour permettre l'introduction des composants constitutifs de la composition dans la chambre interne du réservoir, le réservoir comporte un orifice réalisé dans la paroi cylindrique.

Dans des modes particuliers de réalisation, la tige de l'ensemble mélangeur est un corps allongé, d'aspect lisse à l'extérieur, et présente une section transversale de forme complémentaire à la forme de la section transversale du canal interne longitudinal de la tige filetée de l'ensemble piston. La poignée de l'ensemble mélangeur est fixement solidaire de la tige de l'ensemble mélangeur. L'opérateur doit alors appliquer un mouvement de translation, suivant l'axe longitudinal, et un mouvement de rotation, autour de l'axe longitudinal, à la poignée de l'ensemble mélangeur pour déplacer en translation et en rotation la palette de mélange.

Par « solidaire », on entend que les pièces sont liées l'une à l'autre par une liaison autorisant un mouvement relatif d'une pièce par rapport à l'autre. Par « solidaire fixement », on entend que les pièces sont mutuellement liées de manière fixe, c'est-à-dire qu'un mouvement relatif entre elles est impossible.

Dans des modes particuliers de réalisation, le canal interne longitudinal de la tige filetée de l'ensemble piston présente une nervure hélicoïdale intérieure. La tige de l'ensemble mélangeur est un corps allongé, présentant une rainure hélicoïdale extérieure, ladite rainure hélicoïdale extérieure coopérant avec la nervure hélicoïdale intérieure du canal interne longitudinal de la tige filetée de l'ensemble piston. La poignée de l'ensemble mélangeur est solidaire de la tige de l'ensemble mélangeur. L'opérateur doit alors appliquer uniquement un mouvement de translation, selon l'axe longitudinal, à la poignée de l'ensemble mélangeur pour déplacer en translation et en rotation la palette de mélange de l'ensemble mélangeur. L'engagement de la rainure hélicoïdale de la tige dans la nervure hélicoïdale du canal interne longitudinal de la tige filetée entraine en translation et en rotation ladite tige, et en conséquence la translation et la rotation de la palette de mélange dans le réservoir.

Dans des modes particuliers de réalisation, la tige de l'ensemble mélangeur est un corps allongé, présentant une rainure hélicoïdale extérieure. Le canal interne longitudinal de la tige filetée de l'ensemble piston présente un aspect lisse et est de section transversale supérieure à la section transversale de la tige de l'ensemble mélangeur. La cavité traversante de la poignée de l'ensemble piston présente une section transversale inférieure à la section transversale du canal interne longitudinal de la tige filetée de l'ensemble piston et présente une nervure hélicoïdale intérieure, la rainure hélicoïdale extérieure coopérant avec la nervure hélicoïdale intérieure de la cavité traversante de la poignée de l'ensemble piston. La poignée de l'ensemble mélangeur est solidaire de la tige de l'ensemble mélangeur. L'opérateur doit alors appliquer uniquement un mouvement de translation, selon l'axe longitudinal, à la poignée de l'ensemble mélangeur pour déplacer en translation et en rotation la palette de mélange de l'ensemble mélangeur. L'engagement de la rainure hélicoïdale extérieure de la tige de l'ensemble mélangeur dans la nervure hélicoïdale intérieure de la cavité traversante de la poignée de l'ensemble piston entraine en translation et en rotation ladite tige de l'ensemble mélangeur, et en conséquence la translation et la rotation de la palette de mélange dans le réservoir.

Dans des modes particuliers de réalisation, pour réduire les mouvements répétitifs de va et vient de l'ensemble mélangeur pour l'opérateur, le dispositif comporte un organe de rappel élastique, par exemple un ressort, configuré pour ramener l'ensemble mélangeur vers une position initiale dans laquelle la palette de mélange de l'ensemble mélangeur est contre la tête de piston de l'ensemble piston.

Dans des modes particuliers de réalisation, pour éviter que la palette de mélange se déplace dans la chambre interne du réservoir pendant la phase d'injection, la palette de mélange est solidaire fixement de la tête de piston, pendant ladite phase d'injection. Dans des formes particulières de réalisation, pour rendre solidaire fixement la palette de mélange et la tête de piston, la palette de mélange de l'ensemble mélangeur comporte un manchon, et la tête de piston de l'ensemble piston comporte un logement apte à recevoir le manchon par emboitement.

Dans des modes particuliers de réalisation, pour éviter que la tige de l'ensemble mélangeur n'entrave les mouvements de l'opérateur lorsqu'il est en phase d'injection, la tige de l'ensemble mélangeur est amovible.

Dans des exemples particuliers de réalisation, la tige de l'ensemble mélangeur comporte, au niveau de sa seconde extrémité longitudinale, un filetage. Le manchon de la palette de mélange comporte une cavité centrale taraudée. Le filetage de la tige et le taraudage de la cavité centrale coopère ensemble. La tige de l'ensemble mélangeur peut ainsi être dévissée de la palette de mélange et être extraite hors de l'ensemble piston et donc hors du dispositif.

Dans des modes particuliers de réalisation, pour éviter que la tige de l'ensemble mélangeur n'entrave les mouvements de l'opérateur lorsqu'il est en phase d'injection, la tige de l'ensemble mélangeur présente une zone de fragilité permettant d'amorcer la rupture de ladite tige.

Dans des modes particuliers de réalisation, pour faciliter la préhension du dispositif par l'opérateur, le dispositif comportant un organe de préhension, de type poignée. Dans des exemples particuliers de réalisation, ledit organe de préhension peut être formé par deux demi-coques assemblées l'une à l'autre, venant entourer le réservoir au niveau de sa paroi cylindrique. Les première et seconde extrémités du réservoir ne sont pas emprisonnées par les deux demi-coques.

Dans des exemples particuliers de réalisation, ledit organe de préhension peut être formé par deux demi-coques assemblées l'une à l'autre, venant entourer le réservoir, au moins au niveau de sa première extrémité, et une portion de la tige filetée de l'ensemble piston.

### Brève description des figures

L'invention sera mieux comprise à la lecture de la description suivante, donnée à titre d'exemple nullement limitatif, et faite en se référant aux figures suivantes :
La figure 1 illustre une vue en coupe d'un exemple de dispositif pour le mélange et l'injection d'une composition selon l'invention ;
La figure 2 représente deux variantes d'un exemple de réservoir du dispositif pour le mélange et l'injection d'une composition ;
La figure 3 représente deux autres exemples de réservoir du dispositif pour le mélange et l'injection d'une composition ;
La figure 4 représente une vue en perspective d'une partie d'un ensemble piston du dispositif pour le mélange et l'injection d'une composition ;
La figure 5 illustre une vue en perspective d'un exemple d'une tête de piston de l'ensemble piston du dispositif pour le mélange et l'injection d'une composition ;
La figure 6 est une vue de face de la tête de piston de l'ensemble piston du dispositif pour le mélange et l'injection d'une composition ;
La figure 7 est une vue en perspective d'un exemple d'un ensemble mélangeur du dispositif pour le mélange et l'injection d'une composition ;
La figure 8 est une vue en perspective d'une partie d'un ensemble mélangeur du dispositif pour le mélange et l'injection d'une composition ;
La figure 9 représente une vue en coupe du dispositif pour le mélange et l'injection d'une composition de la figure 1, pendant une phase de mélange ;
La figure 10 représente une vue en coupe du dispositif pour le mélange et l'injection d'une composition de la figure 1, avant une phase d'injection ;
La figure 11 représente une vue en coupe du dispositif pour le mélange et l'injection d'une composition de la figure 1, pendant la phase d'injection ;
La figure 12 représente une vue en coupe d'une variante de réalisation du dispositif pour le mélange et l'injection d'une composition.

Dans ces figures, des références numériques identiques d'une figure à l'autre désignent des éléments identiques ou analogues. Par ailleurs, pour des raisons de clarté, les dessins ne sont pas à l'échelle, sauf mention contraire.

### Description des modes de réalisation

L'invention concerne un dispositif pour le mélange et l'injection d'une composition.

Dans la suite de la description, le dispositif pour le mélange et l'injection d'une composition sera simplement dénommé dispositif 100.

L'invention est décrite dans le contexte particulier d'un de ses domaines d'application préférés dans lequel le dispositif 100 est destiné à être utilisé en milieu médical, notamment pour des opérations de chirurgie osseuse, telles que la cimentoplastie, la vertébroplastie, l'arthroplastie, en vue de consolider un os ou une vertèbre.

Le dispositif selon l'invention permet avantageusement à la fois de recevoir les composants constitutifs de la composition désirée, dite ciment osseux, de réaliser le mélange desdits composants en vue d'obtenir le ciment osseux, et d'injecter le ciment osseux.

Le ciment osseux utilisé au cours des opérations chirurgicales est généralement obtenu, sans que cela soit limitatif, à partir du mélange d'une poudre et d'un liquide.

Dans un exemple de réalisation, la poudre est une poudre de polyméthylméthacrylate (PMMA) et le liquide contient des molécules de méthylméthacrylate (MMA).

Le ciment osseux à l'issue du mélange se présente généralement sous la forme d'une pâte plus ou moins épaisse. Le ciment osseux obtenu présente des propriétés mécaniques et chimiques adaptées et est biocompatible.

La préparation d'un tel ciment osseux est à la portée de l'homme du métier.

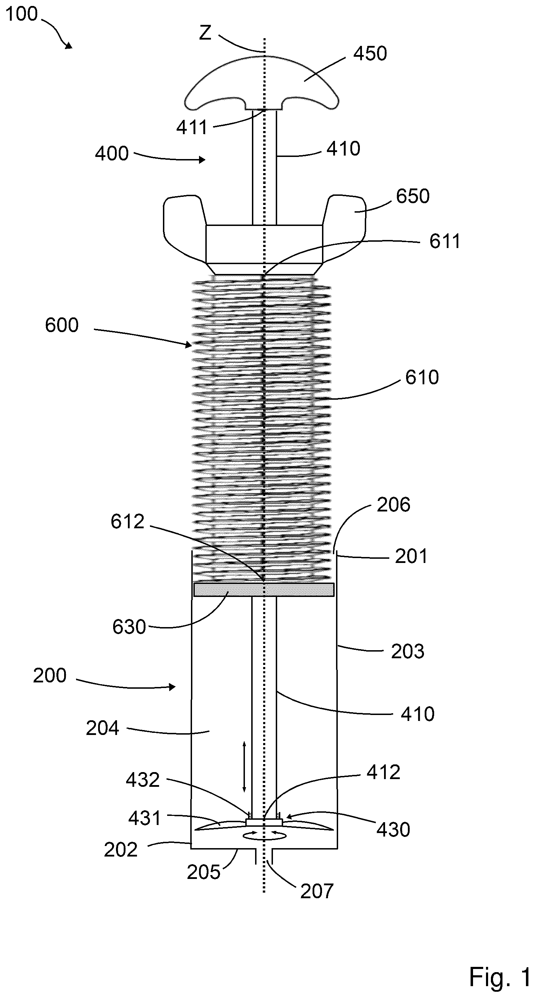

La figure 1 illustre un exemple de dispositif 100 selon l'invention.

Le dispositif 100 comporte un réservoir 200.

Le réservoir 200 présente la forme d'un corps cylindrique creux, de préférence de section circulaire. Le réservoir 200 comporte une paroi cylindrique 203, une première extrémité 201, une seconde extrémité 202, et est d'axe longitudinal Z. Le réservoir 200 délimite une chambre interne 204 dans laquelle des composants peuvent être introduits pour y être mélangés en vue de former le ciment osseux.

Le réservoir 200 comporte, au niveau de sa seconde extrémité 202, un orifice dit de sortie 207. L'orifice de sortie 207 est également en communication avec la chambre interne 204 du réservoir 200.

De préférence, le réservoir 200 présente, au niveau de sa seconde extrémité 202, une paroi 205 comprenant l'orifice de sortie 207, comme illustré sur la figure 1.

L'orifice de sortie 207 peut être équipé d'un connecteur (non représenté) apte à coopérer avec un connecteur complémentaire d'un autre dispositif médical, tel qu'un trocart, une canule, une aiguille, pour transférer le ciment osseux dans la zone à traiter. Le connecteur peut par exemple être du type Luer Lock.

L'orifice de sortie 207 peut être équipé d'un couvercle (non représenté) pour empêcher le ciment osseux de s'écouler via l'orifice de sortie 207 pendant le mélange des composants dans la chambre interne 204 du réservoir 200.

Le réservoir 200 comporte, au niveau de sa première extrémité 201, une ouverture taraudée 206. Le taraudage de l'ouverture est un taraudage intérieur, c'est-à-dire qu'il est réalisé à l'intérieur de l'ouverture. Le taraudage de l'ouverture n'est pas représenté dans les figures 1, 9-11.

L'ouverture taraudée 206 est en communication avec la chambre interne 204 du réservoir 200. L'ouverture taraudée 206 est centrée sur l'axe longitudinal Z du réservoir 200.

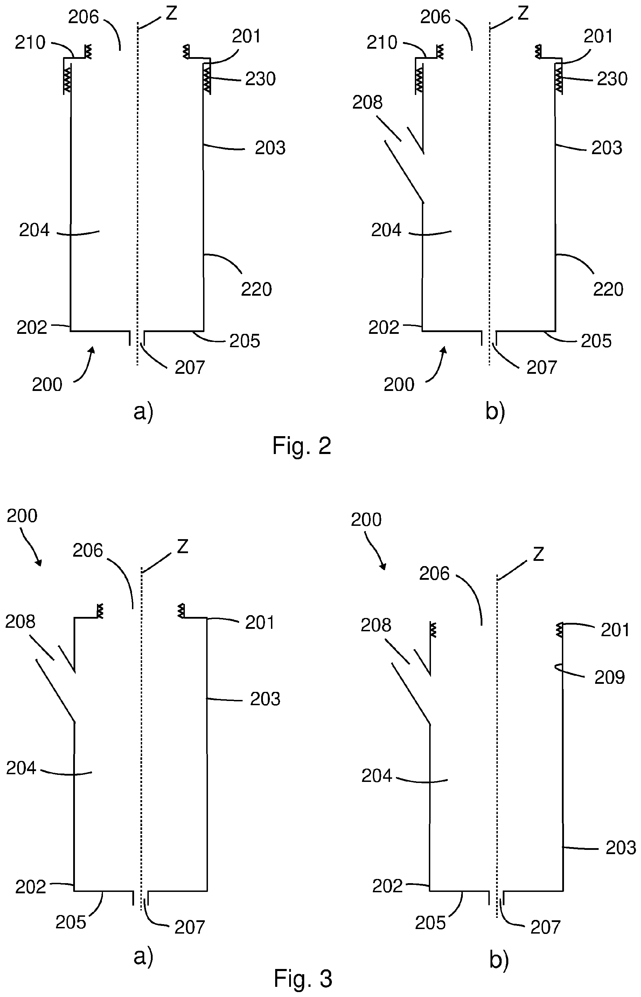

L'ouverture taraudée 206 peut former tout ou partie de la première extrémité 201 du réservoir 200. Dans l'exemple de la vue a) de la figure 3, le réservoir 200 présente, au niveau de sa première extrémité 201, une paroi comprenant l'ouverture taraudée 206. Dans l'exemple de la vue b) de la figure 3, l'ouverture taraudée 206 est formée sur toute la première extrémité 201 du réservoir 200. Le taraudage de l'ouverture est alors réalisé au niveau d'une surface interne 209 de la paroi cylindrique 203 du réservoir 200.

Le réservoir 200 peut être réalisé en un seul bloc, comme illustré sur les vues a) et b) de la figure 3. De préférence, le réservoir 200 comporte un orifice 208 réalisé dans la paroi cylindrique 203 du réservoir 200, pour l'introduction des composants dans la chambre interne 204 du réservoir 200. Ledit orifice 208 peut être équipé d'un connecteur (non représenté) apte à coopérer avec un connecteur complémentaire d'un autre dispositif médical, tel qu'une seringue ou un entonnoir, pour le transfert des composants constituant le ciment osseux dans la chambre interne 204 du réservoir 200. Le connecteur peut par exemple être du type Luer-Lock.

L'orifice 208 peut être équipé d'un couvercle (non représenté) pour empêcher le ciment osseux de s'écouler via l'orifice 208 pendant le mélange des composants dans la chambre interne 204 du réservoir 200.

Le réservoir 200 peut être réalisé en deux parties amovibles 210, 220, comme illustré sur la vue a) de la figure 2, pour permettre l'introduction des composants dans la chambre interne 204 du réservoir 200. Par exemple, une première partie 210 peut comporter l'ouverture taraudée 206 du réservoir et une seconde partie 220 peut comporter l'orifice de sortie 207 du réservoir. La première partie 210 peut par exemple former un couvercle pour la deuxième partie 220. Les deux parties 210, 220 sont assemblées entre elle par des moyens de liaison réversibles, par exemple une liaison de type filetage-taraudage 230. La première partie 210, respectivement la deuxième partie 220, comprend un filetage, respectivement un taraudage, ou inversement, le filetage et le taraudage coopérant pour que les deux parties 210, 220 puissent être assemblées ou désassemblées.

Le réservoir 200, réalisé en deux parties amovibles 210, 220, peut en outre comporter l'orifice 208 réalisé dans la paroi cylindrique 203 du réservoir 200, pour l'introduction des composants dans la chambre interne 204 du réservoir 200, comme illustré sur la vue b) de la figure 3. La seconde partie 220 peut comporter par exemple l'orifice 208. Ledit orifice 208 peut être équipé d'un connecteur (non représenté) apte à coopérer avec un connecteur complémentaire d'un autre dispositif médical, tel qu'une seringue ou un entonnoir, pour le transfert des composants constituant le ciment osseux dans la chambre interne 204 du réservoir 200. Le connecteur peut par exemple être du type Luer-Lock.

L'orifice 208 peut être équipé d'un couvercle (non représenté) pour empêcher le ciment osseux de s'écouler via l'orifice 208 pendant le mélange des composants dans la chambre interne 204 du réservoir 200.

Le réservoir 200 peut être réalisé dans un matériau transparent, par exemple en verre ou en polycarbonate, pour permettre à un opérateur de visualiser le contenu du réservoir 200.

Le dispositif 100 comporte en outre un ensemble mélangeur 400 et un ensemble piston 600.

L'ensemble mélangeur 400 a pour fonction de permettre à l'opérateur de mélanger les composants préalablement introduits dans la chambre interne 204 du réservoir 200 afin d'obtenir le ciment osseux. L'ensemble mélangeur 400 du dispositif 100 est destiné à être utilisé pendant une phase, dite de mélange, comme il sera décrit ultérieurement.

L'ensemble piston 600, quant à lui, a pour fonction de permettre à l'opérateur de transférer le ciment osseux obtenu dans la chambre interne 204 du réservoir 200 hors de ladite chambre interne 204, via l'orifice de sortie 207. L'ensemble piston 600 du dispositif 100 est destiné à être utilisé pendant une phase, dite d'injection, comme il sera décrit ultérieurement. Il est clair de la description que la phase d'injection est réalisée après la phase de mélange.

L'ensemble piston 600 comprend une tige filetée 610, une tête de piston 630 et une poignée 650, comme illustré sur les figures 1, 4 à 6.

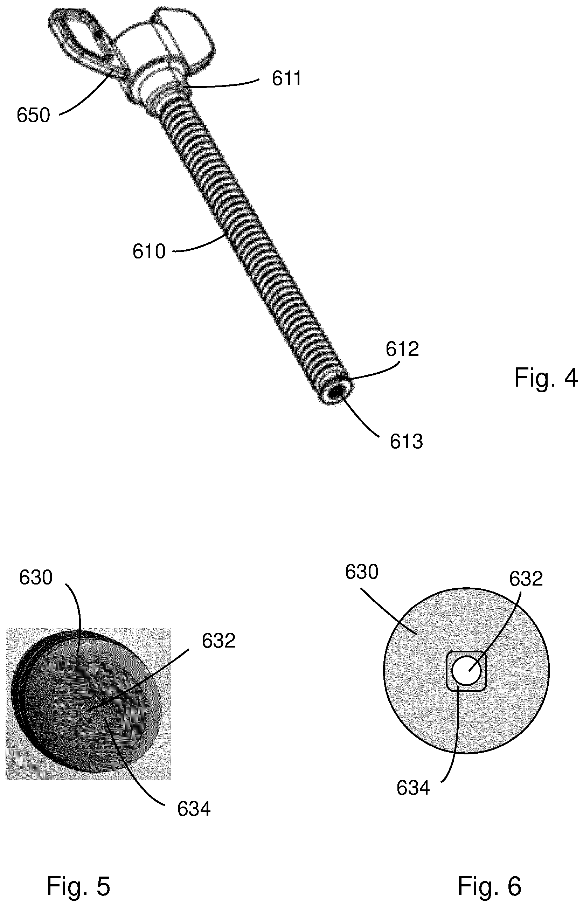

Le filetage de la tige filetée 610 est un filetage extérieur, c'est-à-dire un filetage réalisé au niveau d'une surface extérieure de la tige filetée 610, comme illustrée sur la figure 4. La tige filetée 610 coopère avec l'ouverture taraudée 206 du réservoir 200. La tige filetée 610 présente ainsi un axe longitudinal coaxial avec l'axe longitudinal Z du réservoir 200.

La tige filetée 610 s'étend entre deux extrémités longitudinales 611, 612. Une première extrémité longitudinale 611 est destinée à évoluer hors de la chambre interne 204 du réservoir 200 et une seconde extrémité longitudinale 612 est destinée à évoluer dans ladite chambre interne 204.

La tige filetée 610 est une tige filetée 610 creuse, c'est-à-dire qu'elle comprend un canal interne longitudinal 613 débouchant à ses deux extrémités longitudinales 611, 612.

La poignée 650 de l'ensemble piston 600 comporte une cavité traversante (non représentée sur les figures) dans le prolongement du canal interne longitudinal 613.

La tête de piston 630 de l'ensemble piston 600 est agencée au niveau de la seconde extrémité longitudinale 612 de la tige filetée 610. La tête de piston 630 est agencée dans la chambre interne 204 du réservoir 200 et est configurée pour être mobile en translation, suivant l'axe longitudinal Z du réservoir 200, entre la première extrémité 201 et la seconde extrémité 202 du réservoir 200.

La tige filetée 610 présente ainsi une longueur au moins suffisante pour que la tête de piston 630 évolue entre les deux extrémités 201, 202 du réservoir 200.

La tête de piston 630 est solidaire fixement de la tige filetée 610.

Dans la présente description, on désignera par « solidaire », des pièces liées l'une à l'autre par une liaison autorisant un mouvement relatif d'une pièce par rapport à l'autre. On désignera par « solidaire fixement » des pièces qui sont mutuellement liées de manière fixe, c'est-à-dire qu'un mouvement relatif entre elles est impossible.

La tête de piston 630 se présente sous la forme d'une pièce pleine. Elle présente une section de forme complémentaire à la forme de la section de la paroi cylindrique 203 du réservoir 200. Ainsi, lorsque la paroi cylindrique 203 du réservoir 200 est de section circulaire, la tête de piston 630 présente la forme d'un disque de diamètre sensiblement égal au diamètre interne de la paroi cylindrique 203 du réservoir 200.

Un joint d'étanchéité (non représenté) peut par exemple être disposé au niveau d'une périphérie de la tête de piston 630 afin de combler l'espace entre ladite périphérie de la tête de piston 630 et la paroi cylindrique 203 du réservoir 200 et ainsi d'assurer l'étanchéité entre la tête de piston 630 et la paroi cylindrique 203 du réservoir 200.

La tête de piston 630 présente un orifice traversant 632 (figures 5 et 6) agencé dans la continuité du canal interne longitudinal 613 de la tige filetée 610.

La poignée 650 de l'ensemble piston 600 est agencée au niveau de la première extrémité longitudinale 611 de la tige filetée 610 et est destinée à la manipulation de l'ensemble piston 600 par l'opérateur.

La poignée 650 est solidaire fixement de la tige filetée 610.

La poignée 650 est agencée de sorte à ne pas obturer l'accès au canal interne longitudinal 613 de la tige filetée 610.

Pour utiliser l'ensemble piston 600, l'opérateur manie la poignée 650 de l'ensemble piston 600 en lui appliquant une rotation autour de l'axe longitudinal Z.

En fonction du sens de rotation de ladite poignée 650, la tête de piston 630 se déplace en translation dans le réservoir 200, en s'éloignant ou se rapprochant de la première extrémité 201 dudit réservoir 200.

Ainsi, lorsque la poignée 650 de l'ensemble piston 600 est manœuvrée par l'opérateur dans un premier sens de rotation, dit sens de vissage, par exemple dans le sens des aiguilles d'une montre, la tige filetée 610 est entrainée en rotation autour de son axe longitudinal et entraine, d'une part la translation, selon l'axe longitudinal Z, la tête de piston 630 vers la seconde extrémité 202 du réservoir 200, et d'autre part la rotation de la tête de piston autour de l'axe longitudinal Z. Lorsque la poignée 650 de l'ensemble piston 600 est manœuvrée par l'opérateur dans un deuxième sens de rotation, dit sens de dévissage, c'est-à-dire dans le sens inverse des aiguilles d'une montre, la tige filetée 610 est entrainée en rotation autour de son axe longitudinal et entraine, d'une part la translation la tête de piston 630 vers la première extrémité 201 du réservoir 200 et d'autre part la rotation de la tête de piston autour de l'axe longitudinal Z.

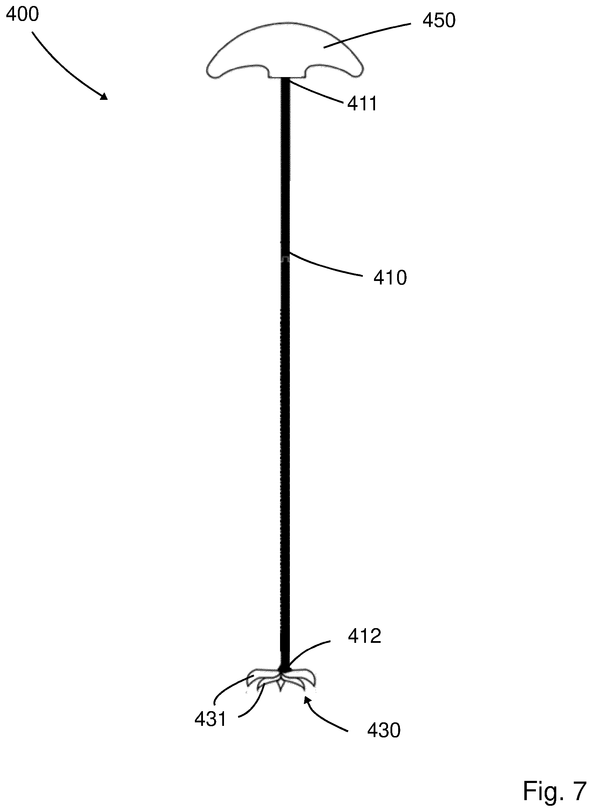

L'ensemble mélangeur 400 comporte une tige 410, une palette de mélange 430 et une poignée 450, comme illustré sur les figures 1 et 7.

La tige 410 traverse la première extrémité 201 du réservoir 200 au niveau de l'ouverture taraudée 206 du réservoir 200.

La tige 410 s'étend entre deux extrémités longitudinales 411, 412. Une première extrémité longitudinale 411 est destinée à évoluer hors de la chambre interne 204 du réservoir 200 et une seconde extrémité longitudinale 412 est destinée à évoluer dans la chambre interne 204 du réservoir 200.

La palette de mélange 430 est agencée au niveau de la seconde extrémité longitudinale 412 de la tige 410. La palette de mélange 430 est agencée dans la chambre interne 204 du réservoir 200 et est configurée pour être mobile d'une part en translation entre la première extrémité 201 et la seconde extrémité 202 du réservoir 200 et d'autre part en rotation autour de l'axe longitudinal Z.

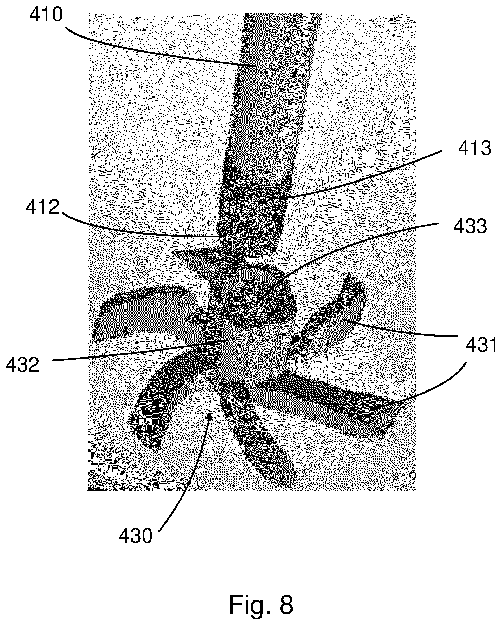

Dans un exemple préféré de réalisation, comme illustrée sur les figures 1, 7 et 8, la palette de mélange 430 comporte une pluralité de pales 431. Chaque pale 431 s'étend depuis la seconde extrémité longitudinale 412 de la tige 410, vers la paroi cylindrique 203 du réservoir 200. Dans l'exemple non limitatif de la figure 8, les pales 431 sont au nombre de six, et présentent une forme incurvée.

Dans une variante (non représentée) de réalisation, la palette de mélange 430 se présente sous la forme d'un disque perforé.

La poignée 450 de l'ensemble mélangeur 400 est agencée, quant à elle, au niveau de la première extrémité longitudinale 411 de la tige 410 et est destinée à la manipulation de l'ensemble mélangeur 400 par l'opérateur.

Selon l'invention, l'ensemble mélangeur 400 est imbriqué dans l'ensemble piston 600. De manière plus précise, la tige 410 de l'ensemble mélangeur 400 s'étend dans le canal interne longitudinal 613 de la tige filetée 610 et dans l'orifice traversant 632 de la tête de piston 630 de l'ensemble piston 600.

La tête de piston 630 de l'ensemble piston 600 est ainsi agencée, dans la chambre interne 204 du réservoir 200, entre la première extrémité 201 du réservoir 200 et la palette de mélange 430 de l'ensemble mélangeur 400.

La poignée 650 de l'ensemble piston 600 est agencée, hors du réservoir 200, entre la première extrémité 201 du réservoir 200 et la poignée 450 de l'ensemble mélangeur 400.

La tige 410 de l'ensemble mélangeur 400 évolue en translation selon l'axe longitudinal Z et en rotation autour dudit axe longitudinal dans le canal interne longitudinal 613 de la tige filetée 610 de l'ensemble piston 600 et dans l'orifice traversant 632 de la tête de piston 630 de l'ensemble piston 600.

La tige 410 de l'ensemble mélangeur 400 présente avantageusement une longueur suffisante pour que, lorsque la tête de piston 630 de l'ensemble piston 600 est au niveau de la première extrémité 201 du réservoir 200, l'opérateur puisse manipuler la poignée 450 de l'ensemble mélangeur 400 de sorte que la palette de mélange 430 de l'ensemble mélangeur 400 puisse évoluer dans la chambre interne 204 du réservoir 200, entre la tête de piston 630 et la seconde extrémité 202 du réservoir 200, comme illustré sur les figure 1, 9 et 10.

La tige 410 de l'ensemble mélangeur 400 présente ainsi une longueur correspondant au moins à la longueur du réservoir 200 et à la longueur de la tige filetée 610 de l'ensemble piston 600.

Ainsi, lors de l'actionnement de l'ensemble mélangeur 400, la palette de mélange 430 de l'ensemble mélangeur 400 peut évoluer dans la chambre interne 204 du réservoir 200, entre la tête de piston 630 et la seconde extrémité 202 du réservoir 200. Lorsque la palette de mélange 430 de l'ensemble mélangeur 400 est positionnée contre la tête de piston 630 de l'ensemble piston 600, l'ensemble mélangeur est dans une position dite initiale.

Dans une première version de réalisation du dispositif 100, comme illustré sur la figure 1, la tige 410 de l'ensemble mélangeur 400 est un corps allongé, d'aspect lisse à l'extérieur. Elle présente une section transversale de forme complémentaire à la forme de la section transversale du canal interne longitudinal 613 de la tige filetée 610 de l'ensemble piston 600 et de l'orifice traversant 632 de la tête de piston 630 de l'ensemble piston 600. Ainsi, dans cette première version, le canal interne longitudinal 613 de la tige filetée 610 de l'ensemble piston 600 permet de guider en translation la tige 410 de l'ensemble mélangeur 400 le long de l'axe longitudinal Z, tout en autorisant sa rotation autour dudit axe longitudinal.

La poignée 450 de l'ensemble mélangeur 400 est, quant à elle, solidaire fixement de la tige 410 de l'ensemble mélangeur 400.

Dans cette première version, l'opérateur doit appliquer un mouvement de translation, suivant l'axe longitudinal Z, et un mouvement de rotation, autour de l'axe longitudinal Z, à la poignée 450 de l'ensemble mélangeur 400 pour déplacer en translation et en rotation la palette de mélange 430.

Dans une deuxième version (non représentée) du dispositif 100, la tige 410 de l'ensemble mélangeur 400 est un corps allongé, présentant une rainure hélicoïdale extérieure, c'est-à-dire une rainure hélicoïdale formée sur une surface extérieure du corps allongé. Le canal interne longitudinal 613 de la tige filetée 610 de l'ensemble piston 600 présente quant à lui une nervure hélicoïdale intérieure. La rainure hélicoïdale extérieure de la tige 410 de l'ensemble mélangeur 400 coopère avec la rainure hélicoïdale intérieure du canal interne longitudinal 613 de la tige filetée 610 de l'ensemble piston 600. Lorsque la cavité traversante de la poignée 650 de l'ensemble piston 600 présente une section transversale égale à la section transversale du canal interne longitudinal 613 de la tige filetée 610 de l'ensemble piston 600, ladite cavité traversante présente une nervure hélicoïdale intérieure dans le prolongement de la nervure hélicoïdale intérieure du canal interne longitudinal 613. Lorsque la cavité traversante de la poignée 650 de l'ensemble piston 600 présente un diamètre supérieur au diamètre du canal interne longitudinal 613 de la tige filetée 610 de l'ensemble piston 600, ladite cavité traversante ne présente pas de nervure hélicoïdale intérieure dans le prolongement de la nervure hélicoïdale intérieure du canal interne longitudinal 613.

Il est également envisageable que le corps allongé de la tige 410 de l'ensemble mélangeur 400 présente une nervure hélicoïdale extérieure, c'est-à-dire une nervure hélicoïdale formée sur une surface extérieure du corps allongé. Le canal interne longitudinal 613 de la tige filetée 610 de l'ensemble piston 600 présente quant à lui une rainure hélicoïdale intérieure.

La poignée 450 de l'ensemble mélangeur 400 est solidaire de la tige 410 de l'ensemble mélangeur 400. Un organe de liaison entre la poignée 450 et la tige 410 de l'ensemble mélangeur 400 permet à la tige 410 de l'ensemble mélangeur 400 de bouger en rotation autour de l'axe longitudinal Z par rapport à la poignée 450.

Dans cette deuxième version, l'opérateur doit alors appliquer uniquement un mouvement de translation, selon l'axe longitudinal Z, à la poignée 450 de l'ensemble mélangeur 400 pour déplacer en translation et en rotation la palette de mélange 430 de l'ensemble mélangeur 400. L'engagement de la rainure hélicoïdale de la tige 410 dans la nervure hélicoïdale du canal interne longitudinal 613 de la tige filetée 610 entraine en translation et en rotation ladite tige 410, et en conséquence la translation et la rotation de la palette de mélange 430 dans le réservoir 200.

Dans une troisième version (non représentée) du dispositif 100, la tige 410 de l'ensemble mélangeur 400 est un corps allongé, présentant une rainure hélicoïdale extérieure. Le canal interne longitudinal 613 de la tige filetée 610 de l'ensemble piston 600 présente un aspect lisse et est de section transversale supérieure à la section transversale de la tige 410 de l'ensemble mélangeur 400. La cavité traversante de la poignée 650 de l'ensemble piston 600 présente une section transversale inférieure à la section transversale du canal interne longitudinal 613 de la tige filetée 610 de l'ensemble piston 600, et présente une nervure hélicoïdale intérieure. La rainure hélicoïdale extérieure de la tige 410 de l'ensemble mélangeur 400 coopère avec la nervure hélicoïdale intérieure de la cavité traversante de la poignée 650 de l'ensemble piston.

La poignée 450 de l'ensemble mélangeur 400 est solidaire de la tige 410 de l'ensemble mélangeur 400. Un organe de liaison entre la poignée 450 et la tige 410 de l'ensemble mélangeur 400 permet à la tige 410 de l'ensemble mélangeur 400 de bouger en rotation autour de l'axe longitudinal Z par rapport à la poignée 450 de l'ensemble mélangeur 400.

Dans cette troisième version, l'opérateur doit alors appliquer uniquement un mouvement de translation, selon l'axe longitudinal Z, à la poignée 450 de l'ensemble mélangeur 400 pour déplacer en translation et en rotation la palette de mélange 430 de l'ensemble mélangeur 400. L'engagement de la rainure hélicoïdale de la tige 410 dans la nervure hélicoïdale de la cavité traversante de la poignée 650 de l'ensemble piston 600 entraine en translation et en rotation la tige 410 de l'ensemble mélangeur 400, et en conséquence la translation et la rotation de la palette de mélange 430 dans le réservoir 200.

Dans un mode de réalisation de l'invention, le dispositif 100 peut comporter un organe de rappel élastique 700 configuré pour ramener l'ensemble mélangeur vers sa position initiale après chaque actionnement. De préférence, l'organe de rappel est configuré pour exercer une force sur la poignée 450 de l'ensemble mélangeur 400 de sorte à ramener l'ensemble mélangeur vers sa position initiale.

Dans une forme préférée de réalisation, l'organe de rappel élastique est un ressort 700.

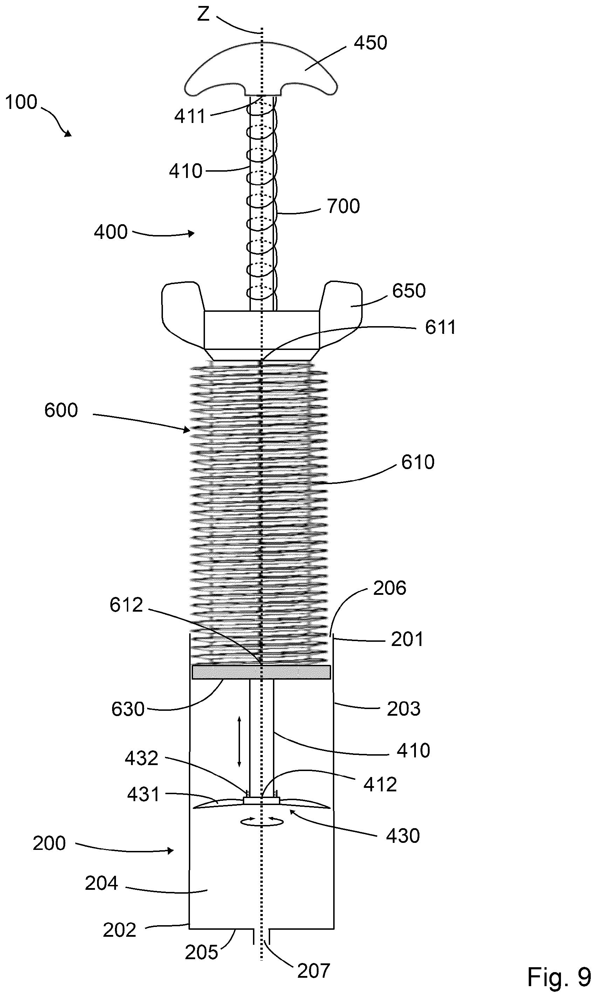

Dans un exemple de réalisation, tel qu'illustré sur la figure 9, le ressort 700 est un ressort hélicoïdal de compression. Ledit ressort hélicoïdal de compression est agencé autour de la tige 410 de l'ensemble mélangeur 400, entre la poignée 450 dudit ensemble mélangeur et la poignée 650 de l'ensemble piston 600. Lorsque l'ensemble mélangeur 400 est en position initiale, le ressort hélicoïdal de compression 700 est en position de repos, c'est-à-dire non comprimée.

Ainsi, en fonctionnement, l'opérateur se saisit de la poignée 450 de l'ensemble mélangeur 400, qui est en position initiale, et applique un mouvement de translation, et éventuellement de rotation selon la version du dispositif 100, à l'ensemble mélangeur 400 pour déplacer la palette de mélange 430 en translation et en rotation dans la chambre interne 204 du réservoir 200, depuis la première extrémité 210 vers la seconde extrémité 220 dudit réservoir. Lors de l'actionnement de l'ensemble mélangeur 400, le ressort hélicoïdal de compression 700 se comprime. L'opérateur peut ensuite relâcher la poignée 450 de l'ensemble mélangeur 400 et l'ensemble mélangeur reprend automatiquement sa position initiale. En d'autres termes, après l'actionnement de l'ensemble mélangeur 400, le ressort hélicoïdal de compression 700 comprimé va élastiquement revenir vers sa position de repos, entraînant avec lui l'ensemble mélangeur vers sa position initiale. Pour revenir vers sa position initiale, la tige 410 de l'ensemble mélangeur 400 suit un mouvement de translation inverse, et éventuellement de rotation inverse selon la version du dispositif 100. L'opérateur se ressaisit à nouveau de la poignée 450 de l'ensemble mélangeur 400 pour un nouvel actionnement de l'ensemble mélangeur 400 jusqu'à ce que la phase de mélange soit terminée.

Dans un autre exemple de réalisation (non représenté sur les figures), le ressort 700 est un ressort hélicoïdal de traction. Ledit ressort est logé dans le canal interne longitudinal 613 de la tige filetée 610 de l'ensemble piston 600, et agencé autour de la tige 410 de l'ensemble mélangeur 400. Cet exemple de réalisation est applicable seulement pour la troisième version du dispositif 100, le canal interne longitudinal 613 de la tige filetée 610 de l'ensemble piston 600 étant de section transversale supérieure à la section transversale de la tige 410 de l'ensemble mélangeur 400, délimitant ainsi un passage permettant l'insertion du ressort 700. Une des extrémités du ressort 700 peut par exemple être maintenue fixement à la première extrémité longitudinale 611 de la tige filetée 610, et l'autre extrémité du ressort 700 peut par exemple être maintenue fixement à la palette de mélange 430 de l'ensemble mélangeur. Lorsque l'ensemble mélangeur 400 est en position initiale, le ressort hélicoïdal de traction 700 est en position de repos, c'est-à-dire non étirée.

Ainsi, en fonctionnement, l'opérateur se saisit de la poignée 450 de l'ensemble mélangeur 400, qui est en position initiale, et applique un mouvement de translation à l'ensemble mélangeur 400 pour déplacer la palette de mélange 430 en translation et en rotation dans la chambre interne 204 du réservoir 200, depuis la première extrémité 210 vers la seconde extrémité 220 dudit réservoir. Lors de l'actionnement de l'ensemble mélangeur 400, le ressort hélicoïdal de traction 700 s'étire. L'opérateur peut ensuite relâcher la poignée 450 de l'ensemble mélangeur 400 et l'ensemble mélangeur reprend automatiquement sa position initiale. En d'autres termes, après l'actionnement de l'ensemble mélangeur, le ressort hélicoïdal de traction 700 étiré va élastiquement revenir vers sa position de repos, entraînant avec lui l'ensemble mélangeur vers sa position initiale. Pour revenir vers sa position initiale, la tige 410 de l'ensemble mélangeur 400 va suivre un mouvement de translation inverse et de rotation inverse. L'opérateur se ressaisit à nouveau de la poignée 450 de l'ensemble mélangeur 400 pour un nouvel actionnement de l'ensemble mélangeur 400 jusqu'à ce que la phase de mélange soit terminée.

Le recours à un organe de rappel élastique 700 permet avantageusement de réduire les mouvements répétitifs de va et vient de l'ensemble mélangeur 400 pour l'opérateur, ce qui peut lui éviter ou limiter les troubles musculo-squelettiques (connu sous l'acronyme TMS).

Dans un mode de réalisation de l'invention, pour éviter que la palette de mélange 430 se déplace dans la chambre interne 204 du réservoir 200 pendant la phase d'injection, la palette de mélange 430 peut venir se fixer sur la tête de piston 630. La palette de mélange est préférentiellement fixée sur la tête de piston de sorte à venir obstruer en même temps l'orifice traversant 632 de la tête de piston 630.

Dans une forme préférée de réalisation, la palette de mélange 430 peut venir se fixer sur la tête de piston 630 par emboitement, encliquetage élastique ou vissage.

Dans l'exemple non limitatif représenté sur les figures 5, 6 et 8, la palette de mélange 430 présente un manchon 432, s'étendant axialement, et la tête de piston 630 présente un logement 634 apte à recevoir le manchon 432 par emboitement. Le logement 634 présente une forme et des dimensions adaptées à un emboitement rigide avec le manchon 432. Dans l'exemple illustré sur les figures 5, 6 et 8, le logement 634 de la tête de piston 630 est coaxial avec l'orifice traversant 632 de la tête de piston 630 et de dimension supérieure à la dimension de l'orifice traversant 632. Le logement 634 peut présenter par exemple une forme carrée, comme illustré sur les figures 5 et 6. Le manchon 432 de la palette de mélange 430 est un manchon 432 central et de forme extérieure complémentaire à la forme du logement 634 de la tête de piston 630. Les pales 431 de la palette de mélange 430 sont solidaires fixement du manchon 432.

Pour fixer la palette de mélange 430 sur la tête de piston 630 par emboitement, l'opérateur doit simplement tirer sur la tige 410 de l'ensemble mélangeur 400 jusqu'à ce que le manchon 432 s'engage dans le logement 634 jusqu'à l'emboitement. Le manchon de la palette de mélange vient ainsi en même temps obturer l'orifice traversant 632 de la tête de piston 630.

Dans un mode de réalisation de l'invention, pour éviter que la tige 410 de l'ensemble mélangeur 400 n'entrave les mouvements de l'opérateur lorsqu'il est en phase d'injection, la tige 410 de l'ensemble mélangeur 400 peut être amovible.

Dans un exemple de réalisation, la tige 410 de l'ensemble mélangeur 400 est amovible au niveau de la seconde extrémité longitudinale 412.

Dans une forme de réalisation, comme illustrée sur la figure 8, la tige 410 de l'ensemble mélangeur 400 comporte, au niveau de sa seconde extrémité longitudinale 412, un filetage 413 et le manchon 432 de la palette de mélange 430 comporte une cavité centrale 433 taraudée. Le filetage 413 de la tige 410 et le taraudage de la cavité centrale 433 coopère ensemble. Lorsque l'opérateur souhaite retirer la tige 410 du dispositif 100, l'opérateur doit, au préalable, fixer la palette de mélange 430 à la tête de piston 630, rendant la palette de mélange 430 solidaire fixement de la tête de piston 630. Puis, l'opérateur peut dévisser la tige 410. Lorsque le filetage 413 de la tige 410 est hors du taraudage de la cavité centrale 433 du manchon 432, l'opérateur peut extraire la tige 410 du canal interne longitudinal 613 de la tige filetée 610 de l'ensemble piston 600.

En variante, pour éviter que la tige 410 de l'ensemble mélangeur 400 n'entrave les mouvements de l'opérateur lorsqu'il est en phase d'injection, la tige 410 peut être cassée.

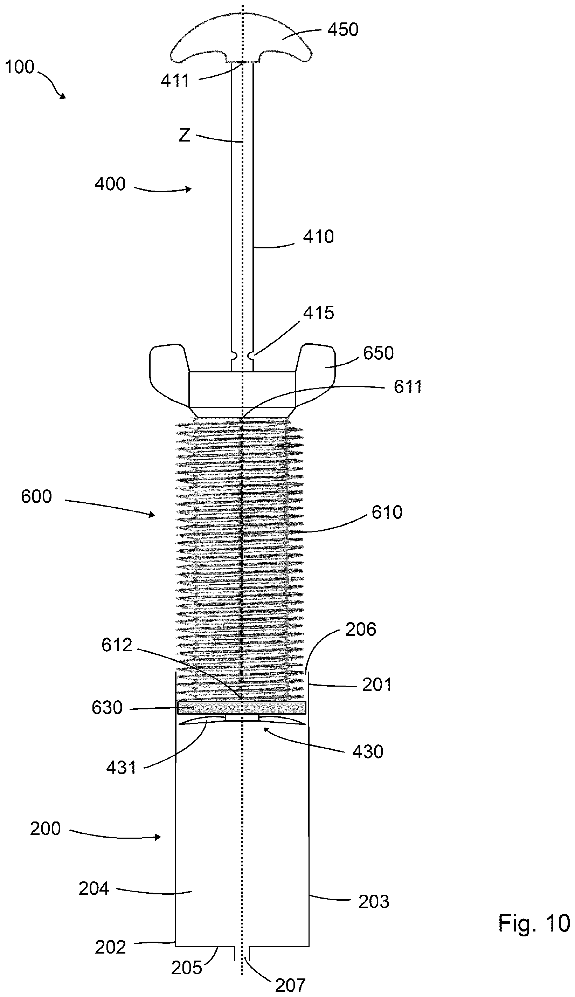

Dans un exemple de réalisation, comme illustré sur la figure 10, la tige 410 de l'ensemble mélangeur 400 peut présenter une zone de fragilité 415 permettant d'amorcer la rupture de ladite tige.

On entend par zone de fragilité, une zone d'affaiblissement mécanique de la tige 410. Dans une forme de réalisation, comme illustré sur la figure 10, la zone de fragilité 415 peut être une zone d'épaisseur réduite.

Dans une autre forme de réalisation (non représentée), la zone de fragilité peut être une encoche.

De préférence, comme illustré sur la figure 10, la zone de fragilité 415 est réalisée sur la tige 410, sur une portion située au niveau de la poignée 650 de l'ensemble piston 600, lorsque la tête de piston 630 et la palette de mélange 430 sont toutes les deux au niveau de la première extrémité 201 du réservoir 200.

La rupture de la zone de fragilité 415 est préférentiellement réalisée par une action manuelle de la part de l'opérateur.

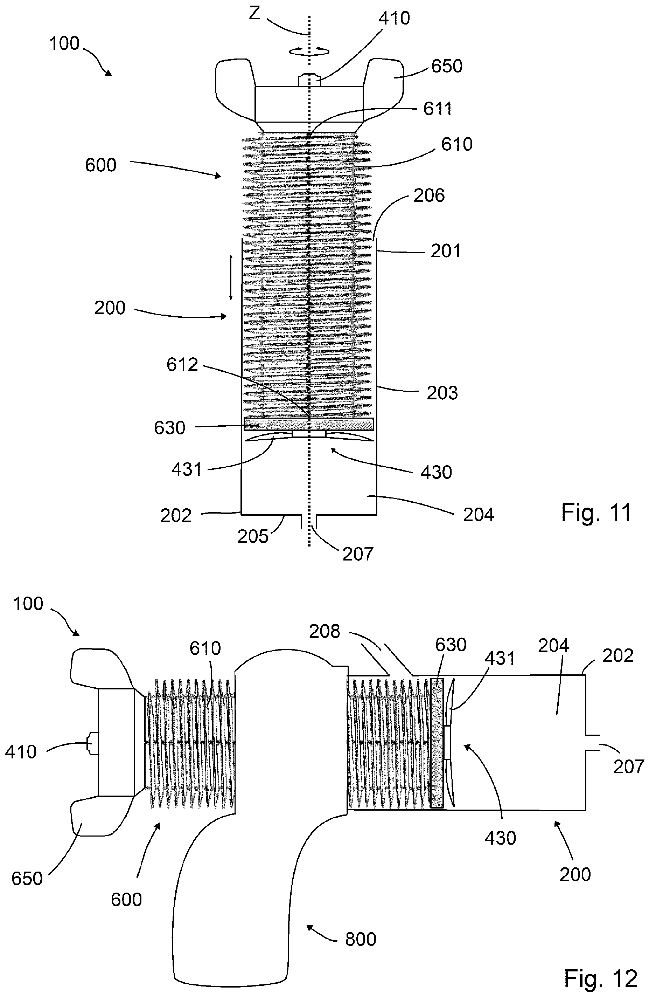

Dans un mode de réalisation de l'invention, illustré sur la figure 12, pour faciliter la manipulation du dispositif 100 par l'opérateur, ledit dispositif comporte un organe de préhension 800, de type poignée.

Dans une forme de réalisation de l'organe de préhension, (non représentée sur les figures) ledit organe de préhension est formé par deux demi-coques assemblées l'une à l'autre, en emprisonnant le réservoir 200 au niveau de sa paroi cylindrique 203. Les première et seconde extrémités 201, 202 du réservoir 200 ne sont pas emprisonnées par les deux demi-coques. Dans cette forme de réalisation, les composants sont introduits soit par l'orifice 208 de la paroi cylindrique 203, soit par la première extrémité 201 du réservoir 200, lorsque le réservoir est en deux parties amovibles 210, 220, comme illustré figure 2.

Dans une autre forme de réalisation, illustrée par la figure 12, ledit organe de préhension est formé par deux demi-coques assemblées l'une à l'autre, en emprisonnant le réservoir 200, au niveau d'au moins sa première extrémité 201, et une portion de la tige filetée 610 de l'ensemble piston 600. Chaque demi-coque peut comporter une empreinte qui, lorsque les deux demi-coques sont assemblées, forme un taraudage coopérant avec le filetage de la tige filetée 610 de l'ensemble piston 600. Les composants sont, dans cette autre forme de réalisation, introduits préférentiellement par l'orifice 208 de la paroi cylindrique 203, comme illustré sur la figure 12.

### Fonctionnement du dispositif 100

Un exemple de fonctionnement du dispositif 100 est à présent décrit.

Dans une phase préalable, les composants sont introduits dans la chambre interne 204 du réservoir.

La tête de piston 630 de l'ensemble piston 600 et la palette de mélange 430 de l'ensemble mélangeur 400 sont chacune placée vers la première extrémité 201 du réservoir 200.

Dans un exemple de mise en œuvre, lorsque le réservoir 200 comporte deux parties amovibles 210, 220, comme illustré sur la vue a) de la figure 2, l'opérateur vient désassembler les deux parties 210, 220 en dévissant. Il introduit ensuite les composants dans la deuxième partie 220, puis assemble à nouveau les deux parties 210, 220 pour refermer le réservoir 200, en revissant les deux parties l'une avec l'autre. Dans un autre exemple de mise en œuvre, lorsque le réservoir 200 comporte, au niveau de sa paroi cylindrique 203, un orifice 208, comme illustré sur la vue b) de la figure 2 et les vues a) et b) de la figure 3, l'opérateur introduit les composants à travers cet orifice 208.

Une fois les composants introduits dans la chambre interne 204 du réservoir, la phase de mélange peut commencer.

L'opérateur se saisit de la poignée 450 de l'ensemble mélangeur 400 et applique un mouvement de translation, et éventuellement de rotation selon la version du dispositif 100, à l'ensemble mélangeur 400 pour déplacer la palette de mélange 430 en translation et en rotation dans la chambre interne 204 du réservoir 200, entre la première extrémité 210 et la seconde extrémité 220 dudit réservoir, comme illustré sur les figures 1 et 9. Le déplacement de la palette de mélange 430 dans la chambre interne 204 du réservoir 200 permet ainsi de mélanger les composants entre eux jusqu'à la constitution du ciment osseux.

La tête de piston 630 de l'ensemble piston 600 est quant à elle restée en position, au niveau de la première extrémité 201 du réservoir 200, pendant toute la phase de mélange.

Lorsque la phase de mélange est terminée, l'opérateur ramène la palette de mélange 430 jusqu'à la première extrémité 201 du réservoir 200 et vient fixer la palette de mélange 430 avec la tête de piston 630 de l'ensemble piston 600 pour les rendre solidaire fixement.

Dans un exemple de mise en œuvre, l'opérateur tire sur la poignée 450 de l'ensemble mélangeur 400 jusqu'à ce que le manchon 432 de la palette de mélange 430 s'emboite dans le logement 634 de la tête de piston 630 de l'ensemble piston 600, comme illustré sur la figure 10.

Lorsque la palette de mélange 430 est solidaire fixement de la tête de piston 630 de l'ensemble piston 600, l'opérateur peut, selon la version du dispositif, soit casser la tige 410 de l'ensemble mélangeur 400, au niveau de sa zone de fragilité 415, soit dévisser la tige 410 de l'ensemble mélangeur 400 du manchon 432 de la palette de mélange 430.

La phase d'injection peut alors commencer. L'opérateur se saisit de la poignée 650 de l'ensemble piston 600 et la tourne dans le sens des aiguilles d'une montre pour visser la tige filetée dans l'ouverture taraudée du réservoir, entrainant le déplacement en translation de la tête de piston 630 et de la palette de mélange 430 vers la seconde extrémité 202 du réservoir 200, poussant le ciment osseux obtenu à travers l'orifice de sortie 207 du réservoir 200.

Grâce à l'engagement de tige filetée 630 de l'ensemble piston 600 dans l'ouverture taraudée 206 du réservoir 200, le dispositif 100 permet avantageusement d'injecter aisément le ciment osseux, même lorsque celui-ci présente une forte viscosité, sans se rompre.

La description ci-avant illustre clairement que par ses différentes caractéristiques et leurs avantages, la présente invention atteint les objectifs qu'elle s'était fixés.

En particulier, elle fournit un dispositif qui permet à la fois de réaliser le mélange de composants en vue d'obtenir une composition, et de l'injecter, avec une grande pression, sans manipulation excessive de la part de l'opérateur.

Bien que l'invention ait été décrite dans le cas d'une utilisation dans le domaine médical, rien n'exclut cependant de l'utiliser dans d'autres domaines d'application. Ainsi, il est par exemple envisageable d'utiliser le dispositif dans le domaine du bâtiment, pour la réparation de fissures.

## Revendications

1. Dispositif (100) pour le mélange et l'injection d'une composition comprenant :
- un réservoir (200) comportant une paroi cylindrique (203), une première extrémité (201) et une seconde extrémité (202), délimitant une chambre interne (204), et d'axe longitudinal (Z),
le réservoir comportant, au niveau de sa première extrémité (201), une ouverture taraudée (206),
le réservoir comportant, au niveau de sa seconde extrémité (202), un orifice de sortie (207),
- un ensemble piston (600) comportant une tige filetée (610), une poignée (650) située à une première extrémité (611) de la tige filetée et une tête de piston (630) située à une seconde extrémité (612) de la tige filetée,
la tige filetée (610) :
∘ coopérant avec l'ouverture taraudée (206) du réservoir (200),
∘ s'étendant selon l'axe longitudinal (Z) du réservoir (200),
∘ comportant un canal interne longitudinal (613) débouchant à ses deux extrémités longitudinales (611, 612),
la tête de piston (630) :
∘ étant agencée dans la chambre interne (204) du réservoir (200),
∘ ayant une section de forme complémentaire à la forme de la section de la paroi cylindrique (203) du réservoir (200),
∘ comportant un orifice traversant (632) dans la continuité du canal interne longitudinal (613) de la tige filetée (610),
la poignée (650) comportant une cavité traversante dans le prolongement du canal interne longitudinal (613) de la tige filetée (610),
- un ensemble mélangeur (400) comportant une tige (410), une poignée (450) située à une première extrémité (411) de la tige et une palette de mélange (430) située à une seconde extrémité (612) de la tige,
la tige (410) de l'ensemble mélangeur (400) s'étendant dans le canal interne longitudinal (613) de la tige filetée (610) et dans l'orifice traversant (632) de la tête de piston (630) de l'ensemble piston (600),
la palette de mélange (430) étant agencée dans la chambre interne (204) du réservoir (200),
**caractérisé en ce que** la tige (410) de l'ensemble mélangeur (400) est un corps allongé présentant une rainure hélicoïdale extérieure, et **en ce que** :
- le canal interne longitudinal (613) de la tige filetée (610) de l'ensemble piston (600) présente une nervure hélicoïdale intérieure, la rainure hélicoïdale extérieure coopérant avec la nervure hélicoïdale intérieure du canal interne longitudinal (613) de la tige filetée (610) de l'ensemble piston (600), ou
- le canal interne longitudinal (613) de la tige filetée (610) de l'ensemble piston (600) présente un aspect lisse et est de section transversale supérieure à la section transversale de la tige (410) de l'ensemble mélangeur (400), la cavité traversante de la poignée (650) de l'ensemble piston (600) présente une section transversale inférieure à la section transversale du canal interne longitudinal (613) de la tige filetée (610) de l'ensemble piston (600) et présente une nervure hélicoïdale intérieure, la rainure hélicoïdale extérieure coopérant avec la nervure hélicoïdale intérieure de la cavité traversante de la poignée (650) de l'ensemble piston (600).

2. Dispositif (100) selon la revendication 1 dans lequel le réservoir est réalisé en deux parties amovibles (210, 220), une première partie (210) comportant l'ouverture taraudée (206) du réservoir (200) et une seconde partie (220) comportant l'orifice de sortie (207) du réservoir (200), la première partie (210) formant un couvercle pour la deuxième partie (220).

3. Dispositif (100) selon l'une des revendications précédentes dans lequel le réservoir (200) comporte un orifice (208) réalisé dans la paroi cylindrique (203) pour l'introduction des composants constitutifs de la composition dans la chambre interne (204) du réservoir (200).

4. Dispositif (100) selon l'une des revendications précédentes dans lequel la palette de mélange (430) de l'ensemble mélangeur (400) est solidaire fixement de la tête de piston (630), pendant une phase d'injection.

5. Dispositif (100) selon la revendication précédente dans lequel la palette de mélange (430) de l'ensemble mélangeur (400) comporte un manchon (432), et la tête de piston (630) de l'ensemble piston (600) comporte un logement (634) apte à recevoir le manchon (432) par emboitement.

6. Dispositif (100) selon l'une des revendications précédentes dans lequel la tige (410) de l'ensemble mélangeur (400) est amovible.

7. Dispositif (100) selon l'une des revendications précédentes dans lequel la tige (410) de l'ensemble mélangeur (400) présente une zone de fragilité (415) permettant d'amorcer la rupture de ladite tige.

8. Dispositif (100) selon l'une des revendications précédentes comportant un organe de préhension (800), de type poignée.

9. Dispositif (100) selon l'une des revendications précédentes comportant un organe de rappel élastique (700) configuré pour ramener l'ensemble mélangeur (400) vers une position initiale dans laquelle la palette de mélange (430) de l'ensemble mélangeur (400) est contre la tête de piston (630) de l'ensemble piston (600).
